(19) Europäisches Patentamt
European Patent Office
Office européen des brevets

(11) Veröffentlichungsnummer: **0 008 472**
**B1**

(12) # EUROPÄISCHE PATENTSCHRIFT

(45) Veröffentlichungstag der Patentschrift: **21.10.81**

(51) Int. Cl.³: **C 07 D207/26**, G 03 C 1/52

(21) Anmeldenummer: **79200439.2**

(22) Anmeldetag: **09.08.79**

(54) **Lichtempfindliche Benzoldiazoniumsalze und Diazotypiematerial unter Verwendung derselben.**

(30) Priorität: **22.08.78 CH 8923/78**

(43) Veröffentlichungstag der Anmeldung: **05.03.80 (Patentblatt 80/05)**

(45) Bekanntmachung des Hinweises auf die Patenterteilung: **21.10.81 Patentblatt 81/42**

(84) Benannte Vertragsstaaten:
**AT BE CH DE FR GB IT NL SE**

(56) Entgegenhaltungen:
**CH - A - 451 701**
**FR - A - 1 350 324**
**FR - A - 1 398 485**
**FR - A - 1 476 460**
**FR - A - 1 543 870**
**FR - A - 2 101 241**
**US - A - 3 868 255**

(73) Patentinhaber: **Aerni-Leuch Ag**
**Zieglerstrasse 34**
**CH-3000 Bern Kanton Bern (CH)**

(72) Erfinder: **Baumann, Niklaus, Dr.**
**Rue du Champ 12**
**CH-1723 Marly Kanton Fribourg (CH)**

(74) Vertreter: **Bovard, Fritz Albert et al**
**Bovard & Cie. Patentanwälte VSP-Rechtsanwälte**
**Optingenstrasse 16**
**CH-3000 Bern 25 (CH)**

**EP 0 008 472 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Lichtempfindliche Benzoldiazoniumsalze und Diazotypiematerial unter Verwendung derselben

Die Erfindung betrifft lichtempfindliche Benzoldiazoniumsalze, die geeignet sind zur Herstellung von Diazotypiematerial, insbesondere Einkomponenten-Diazotypiematerial, mit verbesserter Wasserfestigkeit und ausreichender Entwicklungsgeschwindigkeit.

In der Praxis haben sich für Einkomponenten-Materialien die Salze des 4-Arylmercapto-2,5-dialkoxy-1-diazobenzols, insbesondere 1-Diazo-2,5-dimethoxy-4-p-tolylmercapto-benzol und 2,5-Diäthoxy-1-diazo-4-p-tolylmercaptobenzol bewährt. Diese Verbindungen sind im Fiat Final Report 813, Seiten 138 und 137 und in der holländischen Patentschrift Nr. 51 536 erwähnt. Verbindungen dieses Typs geben mit den herkömmlichen Phloroglucin-Entwicklern schwarze Azofarbstoffe von guter Lichtechtheit und sind in bezug auf Nebenfarbdichten unkritisch. Der Einsatz der erwähnten Verbindungen bleibt aber aufgrund der mässigen Lichtempfindlichkeit und langsamen Kupplungskinetik in Gegenwart der gebräuchlichen schwach sauren Entwicklern auf den Gebrauch in langsam kopierenden Papieren beschränkt. Eine wesentliche Verbesserung der Lichtempfindlichkeit wurde mit den Verbindungen des Typs 2-Chlor-5-p-chlorphenoxy-4-N,N-dialkylamino-1-diazobenzol, die in der Schweizer Patentschrift 386 841 beschrieben sind, erreicht. Allerdings zeigen diese Verbindungen eine langsame Kupplungsgeschwindigkeit und sind deshalb beschränkt anwendbar. Dieser Nachteil ist mitentscheidend, dass sich diese Verbindungen zum Einsatz in transparenten Diazotypiematerialien nicht eignen, da aufgrund des gegenüber opaken Materialien höheren Diazoniumsalzgehaltes keine vollständige Entwicklung in einem Arbeitsgang mit sauren Entwicklern zustandekommt. Dieser Nachteil wurd durch Diazoniumsalze des Typs 4-Arylmercapto-1-diazo-5-methoxy-2-N-methyl-N-alkoxycarbonyl-amino-benzol, die in der Schweizer Patentschrift 448 734 beschrieben sind, und durch Diazoniumsalze des Typs 4-Arylmercapto-1-diazo-5-methoxy-2-pyrrolid-(2)-on-(1)-yl-benzol der Schweizer Patentschrift 451 701 oder Verbindungen des Typs 4-Alkylmercapto-1-diazo-5-methoxy-2-pyrrolid-(2)-on-(1)-yl-benzol der Patentschriften CH 448 734 und DE 1 472 798 und Verbindungen, wie 1-Diazo-4-dimethylamino-5-methoxy-2-pyrrolid-2-on-1-yl-benzol der US-PS 3 868 255, merklich verbessert.

Die Erfahrung zeigt, dass die vorgenannten Verbindungen für den Einsatz in Transparentmaterialien hinsichtlich Kupplungsgeschwindigkeit, Lichtechtheit und Wasserfestigkeit immer noch kein Optimum darstellen, insbesondere bei der Verwendung von lackierten Pauspapieren und Folien. Alle in der Praxis verwendeten Lacke, z.B. auf der Basis von partiell hydrolysierten Celluloseestern oder anderen Polymeren mit hydrophilen funktionellen Gruppen beispielsweise Copolymere des Acrylsäuretyps, weisen teilweise hydrophoben Charakter auf. Dadurch wird die Eindringgeschwindigkeit des wässrigen Entwicklers stark beeinflusst. Als Folge manifestiert sich besonders bei schnell laufenden Kopier-Entwicklungsmaschinen eine mangelhafte Farbdichte in den unbelichteten Teilen der Kopie, was zu einem Informationsverlust bei Tochterpausen führt.

Es ist Gegenstand der Erfindung, neue Diazoniumsalze zu beschreiben, welche die Mängel der etwas zu langsamen Entwicklung nicht aufweisen, ohne dass negative Eigenschaften, wie mangelnde Lichtempfindlichkeit, Nebenfarbdichten und schlechte Lichtechtheit der gebildeten Azofarbstoffe auftreten.

Es ist im weiteren Gegenstand der Erfindung, die Wasserfestigkeit der Diazoniumsalze in der Schicht gegenüber wässrigen Entwicklern und die Wasserfestigkeit der nach dem Entwickeln gebildeten Azofarbstoffe gegenüber den Verbindungstypen, die in den Patentschriften CH 448 734, 451 701 und DE 1 472 798 beschrieben wurden, zu verbessern. Diese Eigenschaften bewirken ein deutlich geringeres Ausbluten in der hergestellten Kopie. Dadurch wird eine optimale Strichreproduktion gewährleistet. Im weiteren bewirkt die gute Wasserfestigkeit der gebildeten Azofarbstoffe eine Verbesserung von auf dieser Basis hergestellten Offsetdruckplatten.

Die neuen lichtempfindlichen Benzoldiazonium-Verbindungen gemäss der Erfindung haben die Formel

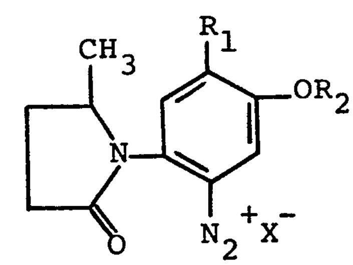

(I)

worin $R_1$ p-Methylphenylthio, Butylthio, Phenylthio oder Benzylthio, $R_2$ Methyl, Aethyl, Alkoxyalkyl mit 1 oder 2 Kohlenstoffatomen in dem Alkyl- und Alkoxyrest oder p-Chlorphenyl und X ein Anion bedeuten.

Bevorzugte Verbindungen der Formel I sind solche, in denen X ein Chlor- Tetrafluoroborat-, Hexafluorophosphat- oder $1/2ZnCl_4^-$-Ion ist.

Als Salze der Verbindungen der Formel I kommen beliebige organische oder anorganische Salze in Frage, wie beispielsweise einfache Salze, wie das Chlorid, oder Sulfat, das Tetrafluoroborat, Hexafluorophosphat, Hexafluoroarsenat, oder als Doppelsalze, wie das Zink-, Eisen-, Cobalt-, Antimon- oder Zinnchlorid-Doppelsalz, wobei das Zinkchlorid-Doppelsalz bevorzugt ist. Als organische Salze seien genannt solche mit organischen Sulfonaten beispielsweise 1,3,6-Trisulfonaphthalin, 1,5-Disulfonaphthalin, 1,6-Disulfonaphthalin.

Besonders bevorzugte Verbindungen der Formel I sind solche, in denen $R_1$ p-Methylphenylthio, $R_2$ Methyl, Aethyl, Methoxyäthyl oder p-Chlorphenyl und X ein Chlor-Tetrafluoroborat-, Hexafluorophosphat oder $1/2ZnCl_4^-$-Ion, oder $R_1$ Butylthio, Phenylthio oder Benzylthio, $R_2$ Methyl und X ein Chlor-, Tetrafluoroborat-, Hexafluorophosphat-, oder $1/2ZnCl_4^-$-Ion, oder $R_1$ p-Methylphenylthio, $R_2$ Methyl, Aethyl, Methoxyäthyl oder p-Chlorphenyl und X ein $1/2ZnCl_4^-$-Ion sind. Insbesondere sind ferner Verbindungen bevorzugt, in denen $R_1$ Butylthio, Phenylthio oder Benzylthio, $R_2$ Methyl und X ein $1/2ZnCl_4^-$-Ion, oder $R_1$ p-Methylphenylthio, $R_2$ Methyl und X ein $1/2ZnCl_4^-$-Ion sind.

Die Komponenten gemäss der Erfindung werden vorteilhaft ausgehend von 3,4-Dichlor-nitrobenzol synthetisiert. Im ersten Reaktionsschritt erfolgt die nukleophile Substitution des Chloratoms in Position 4 durch eine Alkoxy- oder Aryloxy-Gruppe. Anschliessend wird die Nitrogruppe zum entsprecheden Anilin reduziert und nachfolgend mit einem substituierten Butyrolacton unter Abspaltung von Wasser an das Anilin zu einem N-substitutierten Lactam kondensiert. Dieses lässt sich ausserordentlich gut und in allen Fällen am aromatischen Ring in para-Stellung zum Chloratom nitrieren.

Der Ersatz des Chloratoms erfolgt durch nukleophile Substitution mittels Mercaptanen oder Aminen. Diese Reaktionen verlaufen unter relativ milden Bedingungen und geben sehr einheitliche Produkte. Die nachfolgende Reduktion der Nitrogruppe zum Amin ist katalytisch oder mit konventionellen Methoden einfach durchführbar. Insbesondere erweist sich hier der substituierte Butyrolactamring gegenüber dem unsubstituierten Butyrolactamring oder gegenüber einer offenkettigen $-N-C=O$ Verknüpfung als ausserordentlich resistent gegenüber Hydrolyse oder Hydrogenolyse. Die erfindungsgemässen Verbindungen unterscheiden sich daher auch hinsichtlich ihrer Herstellung vorteilhaft gegenüber ähnlichen Verbindungen, wie sie aus dem Stand der Technik bekannt sind.

Das Amin wird in üblicher Weise in das Diazoniumsalz überführt. Sofern 5-methoxysubstituierte Diazoniumsalze angestrebt werden, ist es möglich, die Synthese mit 2-Nitro-anisidin zu beginnen. Bis zu einem gleichen Grundkörper wie in obiger Synthese werden die Stufen Lactonkondensation, Reduktion der Nitrogruppe zum Amin und Ersatz der Aminogruppe durch ein Chlor, mittels Sandmeyer Reaktion, durchlaufen. Im weiteren ist es möglich, das Diazoniumsalz ausgehend von o-Chlor-p-nitrophenol aufzubauen. Dabei wird im ersten Schritt das Phenol alkyliert und anschliessend zum Amin reduziert. Die Alkylierung von p-Nitrophenolen verläuft aber aufgrund des starken minus M-Effektes der Nitrogruppe meist unbefriedigend.

Im folgenden wird die Synthese der in der folgenden Tabelle angegebenen Verbindungen beschrieben.

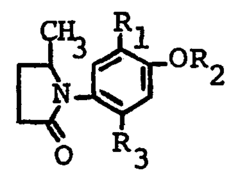

| Verbindung Nr. | $R_1$ | $R_2$ | $R_3$ | Smp. °C | UV-Spektrum[1] nm |
|---|---|---|---|---|---|
| 1 | Cl | $CH_3$ | H | 61 | |
| 2 | Cl | $CH_3$ | $NO_2$ | 150-151 | |
| 3 | 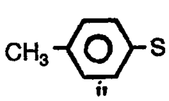 | $CH_3$ | $NO_2$ | 187-188 | |
| 4 | " | $CH_3$ | $NH_2$ | 196 | |
| 5 | " | $CH_3$ | $N_2^{\oplus}$ | — | 368/408s |
| 6 | $CH_3(CH_2)_3S$ | $CH_3$ | $NO_2$ | 113 | |
| 7 | $CH_3(CH_2)_3S$ | $CH_3$ | $NH_2$ | 154 | |

| Verbindung Nr. | $R_1$ | $R_2$ | $R_3$ | Smp. °C | UV-Spektrum[1] nm |
|---|---|---|---|---|---|
| 8 | $CH_3(CH_2)_3S$ | $CH_3$ | $N_2^{\oplus}$ | — | 378/405s |
| 9 | 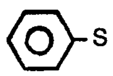 | $CH_3$ | $NO_2$ | 156 | |
| 10 | " | $CH_3$ | $NH_2$ | 142 | |
| 11 | " | $CH_3$ | $N_2^{\oplus}$ | — | 368/405s |
| 12 | 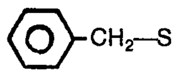 | $CH_3$ | $NO_2$ | 147 | |
| 13 | " | $CH_3$ | $NH_2$ | Oel | |
| 14 | " | $CH_3$ | $N_2^{\oplus}$ | — | 370/405s |
| 15 | Cl | $C_2H_5$ | H | Oel | |
| 16 | Cl | $C_2H_5$ | $NO_2$ | 119-121 | |
| 17 | 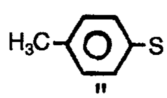 | $C_2H_5$ | $NO_2$ | 141 | |
| 18 | " | $C_2H_5$ | $NH_2$ | 102 | |
| 19 | " | $C_2H_5$ | $N_2^{\oplus}$ | — | 370/410 |
| 20 | Cl | $CH_2CH_2—OCH_3$ | H | Oel | |
| 21 | Cl | $CH_2CH_2—OCH_3$ | $NO_2$ | Oel | |
| 22 | 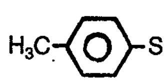 | $CH_2CH_2—OCH_3$ | — | 176 | |
| 23 | " | $CH_2CH_2—OCH_3$ | $NH_2$ | 94 | |
| 24 | " | $CH_2CH_2—OCH_3$ | $N_2^{\oplus}$ | — | 370/408s |
| 25 | Cl | 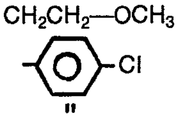 | H | Oel | |
| 26 | Cl | " | $NO_2$ | 140 | |
| 27 | 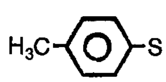 | " | — | 183 | |
| 28 | " | | $NH_2$ | 142 | |
| 29 | " | | $N_2^{\oplus}$ | — | 375/400s |

(1) Die Maxima der Diazoniumsalze wurden in den sensibilisierten Schichten aus Beispiel (III) ermittelt. Die Salze wurden als Zinkchlorid-Doppelsalz eingesetzt.

Verfahren zur Herstellung der Verbindung Nr. 5

a) Verbindung Nr. 1

660 g 3-Chlor-4-methoxyanilin (4,19 M) werden mit 3 ml konz. Schwefelsäure, 500 ml o-Xylol und 462 g γ-Valerolacton (4,19 M) versetzt. Die Lösung wird 6 Stunden unter Rückfluss gehalten, wobei das entstehende Wasser azeotrop abdestilliert wird. Nach beendeter Reaktion entfernt man das Xylol durch Destillation und wäscht mit 3 Portionen von 100 ml Wasser neutral. Aus Methanol umkristallisiert erhält man ein weisses Pulver, das einen Smp. von 61 °C aufweist. Die Ausbeute des praktisch reinen Rohproduktes beträgt 1 107 g oder 95 % der Theorie.

b) Verbindung Nr. 2

210 g Verbindung Nr. 1 (0,876 M) werden in 420 ml Eisessig gelöst, in Mischsäure, bestehend aus 130 ml $HNO_3$ (65 %) und 630 ml Eisessig, bei 100 °C langsam eingetropft. Nach beendetem Zutropfen wird 3 Stunden weitergerührt und nach dem Erkalten auf 5 l Eiswasser gegossen. Die Nitroverbindung fällt im Form bräunlich-gelber Kristalle an. Die Ausbeute nach dem Umkristallisieren aus Methanol beträgt 224 g oder 90 % der Theorie. Smp. 151 °C.

c) Verbindung Nr. 3

96 g Thiokresol (0,77 M) werden mit 50 g Kaliumhydroxid in Dimethylformamid gelöst. Bei 20 °C fügt man 212 g Verbindung Nr. 2 (0,745 M) in Portionen zu und erhöht die Reaktionstemperatur allmählich auf 160 °C. Nach 3 Stunden ist die Reaktion beendigt. Nach dem Erkalten giesst man auf 2 l Eiswasser und filtriert von den Kristallen ab. Der Smp. des aus Essigester umkristallisierten Produktes beträgt 190 °C. Die Ausbeute an reinem Produkt ist 236 g oder 85 % der Theorie.

d) Verbindung Nr. 4

255 g der Verbindung Nr. 3 (0,68 M) werden in 10 200 ml Benzol unter Rühren gelöst und am Rückfluss erhitzt. 2 550 g Fe° (Pulver) frisch aktiviert wird auf einmal beigefügt. Unter Rückfluss fügt man je nach Reaktionsverlauf 0,5-1 ml Wasser pro Stunde bei. Die Reaktion ist nach 5-6 Stunden beendet. Man filtriert heiss über Kieselgur ab und wäscht mit 5 × 500 ml Benzol nach. Nach dem Eindampfen kristallisiert man aus 5 l Methanol um. Ausbeute 196 g (83,66 %), Smp. 196 °C. Die Methode ist beschrieben bei Houben-Weyl, Bd. II/I, S. 398-399. Ebenso können bekannte Reduktionsverfahren, beispielsweise nach Béchamp oder mittels katalytischer Hydrierung, eingesetzt werden.

e) Verbindung Nr. 5

10,4 g der Verbindung Nr. 4 (0,03 M) werden in 100 ml Aceton gelöst und bei 0 °C mit 25 ml 32 %iger Salzsäure versetzt. Bei gleicher Temperatur werden langsam 20 ml 2N-Natriumnitritlösung beigefügt. Nach beendeter Diazotierung wird mit 9 ml 50 %iger Zinkchloridlösung versetzt und das Aceton bei Zimmertemperatur im Vakuum verdampft. Es scheiden sich gelbe Kristalle des Zinkchlorid-Doppelsalzes ab. Ausbeute nach dem Trocknen 13,3 g oder 90 % der Theorie.

In entsprechender Weise, wie vorstehend beschrieben, lassen sich nach grundsätzlich analogen Verfahren die Verbindungen Nrn 6-29 der vorstehenden Tabelle in guten Ausbeuten herstellen.

Wie erwähnt können die erfindungsgemässen Verbindungen als lichtempfindliche Komponente in Diazotypiematerialien verwendet werden. Dieses Diazotypiematerial kann beispielsweise sogenanntes Einkomponenten-Diazotypiematerial, das mit einem flüssigen Entwickler entwickelt wird, sogenanntes Zweikomponenten-Diazotypiematerial, das mit Hilfe von Ammoniakdampf entwickelt wird, oder durch Anwendung von Wärme zu entwickelndes Diazotypiematerial sein. Bevorzugt ist die Verwendung zur Herstellung von Einkomponentenmaterial, wobei ein beliebiger Träger verwendet werden kann, der insbesondere opak oder transparent sein kann, wie beispielsweise Papier, Leinen, Karton, Metallfolie, pigmentierte organische Folie bzw. Transparentpapier, transparente organische Folien, wie beispielsweise Polypropylen, Polyester, Celluloseester und andere.

Wenn das Diazotypiematerial gemäss der Erfindung ein Zweikomponentenmaterial ist, werden vorzugsweise schwach aktive Kuppler, wie 2-Hydroxy-biguanidinonaphtalin oder 3,6-Disulfo-2-hydroxynaphtalin, eingesetzt. Als thermisches Diazomaterial wird ein solches hergestellt, wie es in den BP 815 005 und 983 799 beschrieben ist.

Zur Herstellung einer Druckvorlage kann gekörntes Aluminium oder ein speziell geeignetes Diazopapier, beispielsweise mit Baryt beschichtetes Papier der Qualität « Dryphotomat ». (Papeteries Steinbach & Cie., Malmédy, Belgien) oder Diazobeschichtungskarton (Papierfabrik Cham, Schweiz) verwendet werden. Die Sensibilisierung des Materials erfolgt aus einer wässrigen oder organischwässrigen Lösung in an sich bekannter Weise. Diese kann zusätzlich Stabilisatoren, wie Zitronensäure, Weinsäure, Borsäure, Benzol- und Naphtalinsulfosäuren oder ihre Salze, Metallsalze, beispielsweise Zinkchlorid, Magnesiumchlorid, Nickelsulfat und Alaun enthalten. Ebenso können gemäss der Erfindung Oberflächenverbesserungsmittel, wie kolloidales oder nichtkolloidales Siliciumdioxid, Aluminiumoxid, Reisstärke, Bariumsulfat u. a. sowie Bindemittel, wie Gummiarabicum, Gelatine, Celluloseäther, Stärkederivate, Polyvinylalkohole, Polyacrylsäuren, Polymaleinsäuren und Copolymere davon, sowie Dispersionen von organischen Kunststoffen für den Materialaufbau verwendet werden.

Ferner lassen sich Filme, die eine flache Gradation aufweisen, durch die Verwendung der Dünnschichttechnik oder die Applikation von UV-Absorbern herstellen.

Besonders geeignete Verbindungen sind solche, die eine Benzamido oder eine S-Alkyl-, S-Aralkyl- und S-Arylgruppe in p-Stellung zur Diazogruppe aufweisen. Solche Verbindungen weisen sich durch eine hohe Lichtempfindlichkeit und Kupplungsaktivität aus. Die Photozersetzungsprodukte sind farblos, und die gebildeten Azofarbstoffe zeigen eine sehr gute Lichtechtheit.

Die Herstellung der Diazotypiematerials gemäss der Erfindung wird durch die folgenden Beispiele im einzelnen erläutert.

Entwickler

Die Entwickler für Einkomponentenmaterialien variieren oft in der Zusammensetzung. Sie bestehen aber grundsätzlich aus einem wasserlöslichen organischen Puffersystem, das Kupplungskomponenten, meist Phloroglucin, Resorcin und Acetanilid sowie Netzmittel und Antioxydantien enthält. In der Praxis durchgesetzt haben sich schwach saure Entwickler mit organischen Säure/Base-Paaren, die pKa-Werte von 4-6,5 aufweisen. Die beiden nachfolgend genannten Entwickler werden in den folgenden Beispielen zur Entwicklung verwendet:

Entwickler I ist eine Lösung von
8 g Phloroglucin
0,1 g Acetoanilid
0,3 g « Pluriol 10 800 » (Polyäthylenoxid der BASF, Ludwigshafen, BRD)
8 g Thioharnstoff
2,3 g Benzoesäure
15 g Natriumbenzoat
130 g Natriumformiat
1 000 ml Wasser
Die Lösung zeigt einen pH-Wert von ungefähr 5,8.

Entwickler II ist eine Lösung von
6 g Phloroglucin
4,5 g Resorcin
0,4 g 2-Aethyl-1-hexyl-sulfat «Tergitol-08» (Union Carbide, New York, USA)
0,3 g Natriumbisulfit
15 g Natriumformiat
20 g Dinatriumadipinat
45 g Trinatriumzitrat $2H_2O$
2 g Adipinsäure
1 000 ml Wasser
Die Lösung zeigt einen pH-Wert von ungefähr 6,4.

Anstelle von Phlorogucin oder Resorcin können in schwach sauren Entwicklern auch andere Kupplungskomponenten, wie 2,3-Dihydroxynaphtalin oder 2-Hydroxynaphtalin, verwendet werden.

Zur Sensibilisierung von Trägermaterialien können folgende Grundrezepturen eingesetzt werden :

Lösung A für opake Materialien:
1-2 % Diazoniumsalz
10 g Zitronensäure
3 g Saponin
1 000 ml Wasser

Lösung B für transparente Materialien :
5-7 % Diazoniumsalz
10 g Zitronensäure
3 g Saponin
100-200 ml Isopropanol oder Aceton
800-1 000 ml Wasser.

In diesen Lösungen lassen sich zusätzliche oder andere Komponenten, wie Stabilisatoren, Kieselsäure, Kunststoffdispersionen u.a., verwenden.

Die Herstellung der sensibilisierten Schichten erfolgt nach an sich bekannten Verfahren, so wird beispielsweise bei Papieren oder Filmen die Lösung durch Applikation mittels Auftragswalzen und Abblasen der angeschwemmten Flüssigkeit durch einen Luftmesser bewirkt. Der Auftrag der Lösung auf dem Papier oder Film beträgt etwa 10-16 ml/m$^2$. Das Auftragen der Schicht erfolgt bei Aluminiumplatten oder Offsetplatten auf Papierbasis nach der üblichen Schleudertechnik.

Beispiel I

Ein Diazo-Rohpapier wird sensibilisiert mit Lösung A, die 20 g der Verbindung Nr. 5 gemäss der Erfindung als Zink-chlorid-Doppelsalz enthält (Probe 1). Ein weiteres Papier mit Lösung A, die 20 g 1-Diazo-5-methoxy-2-pyrrolid-2-on-1-yl-4-p-tolylthio-benzol-Zinkchlorid-Doppelsalz (bekannte Vergleichssubstanz) enthält (Probe 2).

Auf beiden Papieren werden je 16 ml Lösung pro m$^2$ Oberfläche appliziert.

Je ein 20 cm breiter Streifen wird auf 10 cm ausbelichtet. Diese Proben werden zur Entwicklung 2 s in Entwicklerlösung II getaucht. Nach dem Trocknen weist das Papier der Probe 1 keinen Farbschleier im ausbelichteten Teil auf. Probe 2 zeigt einen deutlich feststellbaren Farbschleier, der durch das sogenannte Ausbluten zustande kommt. Beide Proben zeigen ähnliche rotbraune Farbtöne. Die

Belichtung durch einen Kodak-20 Stufenkeil (ΔOD = 0,15) zeigt, dass das Papier der Probe 1 etwas schneller gegenüber demjenigen der Probe 2 ist.

Beispiel II

Aehnlich gute Resultate ergeben sich durch den Vergleich der erfindungsgemässen Verbindungen 8, 11 und 29 gegenüber aus dem Stand der Technik bekannten Verbindungen, und zwar:

Verbindung 8 gegenüber 1-Diazo-5-methoxy-4-n-butylthio-2-pyrrolid-2-on-1-yl-benzol-Zinkchlorid-Doppelsalz und

Verbindung 11 gegenüber 1-Diazo-5-methoxy-4-phenylthio-2-pyrrolid-2-on-1-yl-benzol-Zinckchlorid-Doppelsalz, oder

Verbindung 29 gegenüber 1-Diazo-5-p-chlorphenoxy-2-pyrrolid-2-on-1-yl-benzol-Zinkchlorid-Doppelsalz.

Beispiel III

Ein Transparentpapier, das eine hydrophile Lackschicht auf der Basis von Polyvinylacetat aufweist, wird mit je 50 g der Verbindungen 5, 8, 11, 14, 19, 24 und 29 in Lösung B sensibilisiert und durch einen Kodak-20 Stufenkeil (wie in Beispiel I) belichtet und anschliessend mit Entwickler I entwickelt. Es zeigt sich dabei, dass alle Verbindungen ungefähr denselben Reproduktionsumfang ergeben. Die in der Schicht mit Phloroglucin gebildeten Azofarbstoffe zeigen folgende Absorptionsmaxima im elektromagnetischen Absorptionsspektrum:

Verbindung Nr. 5 420/555 <nm>
8 405/545
11 415/546
14 465/550 s
19 410/562
24 416/562
29 408/534

Ein erfindungsgemässes Zweikomponentendiazotypiematerial kann hergestellt werden durch Einverleiben einer Verbindung der Formel I in einen an sich bekannten Schichtaufbau und Beschichten des gewünschten Trägermaterials. Das erfindungsgemässe Material kann auch mit langsam kuppelnden Komponenten, z.B. 7'-Hydroxy-1',2',4,5-naphtimidazol oder 2-Hydroxy-8-biguanidino-naphtalin, wie dies in der deutschen Patentschrift 1 814 283 beschrieben ist, ausgerüstet werden.

**Ansprüche** für die Vertragsstaaten BE, CH, DE, FR, GB, IT, NL, SE

1. Lichtempfindliche Benzoldiazoniumverbindung der Formel

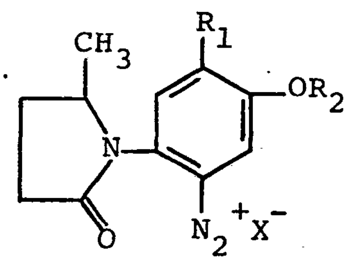

(I)

worin $R_1$ p-Methylphenylthio, Butylthio, Phenylthio oder Benzylthio, $R_2$ Methyl, Aethyl, Alkoxyalkyl mit 1 oder 2 Kohlenstoffatomen in dem Alkyl- und Alkoxyrest oder p-Chlorphenyl und X ein Anion ist.

2. Verbindung der Formel I nach Anspruch 1, worin X ein Chlor-, Tetrafluoroborat-, Hexafluorophosphat- oder $1/2ZnCl_4^-$-Ion ist.

3. Verbindung der Formel I nach Anspruch 1, worin $R_1$ p-Methylphenylthio, $R_2$ Methyl, Aethyl, Methoxyäthyl oder p-Chlorphenyl und X ein Chlor-, Tetrafluoroborat-, Hexafluorophosphat oder $1/2ZnCl_4^-$-Ion ist.

4. Verbindung der Formel I nach Anspruch 1, worin $R_1$ Butylthio, Phenylthio oder Benzylthio, $R_2$ Methyl und X ein Chlor-, Tetrafluoroborat-, Hexafluorophosphat- oder $1/2ZnCl_4^-$-Ion ist.

5. Verbindung der Formel I nach Anspruch 1, worin $R_1$ p-Methylphenylthio, $R_2$ Methyl, Aethyl, Methoxyäthyl oder p-Chlorphenyl und X ein $1/2ZnCl_4^-$-Ion ist.

6. Verbindung der Formel I nach Anspruch 1, worin $R_1$ Butylthio, Phenylthio oder Benzylthio, $R_2$ Methyl und X ein $1/2ZnCl_4^-$-Ion ist.

7. Verbindung der Formel I nach Anspruch 1, worin $R_1$ p-Methylphenylthio, $R_2$ Methyl und X ein $1/2ZnCl_4^-$-Ion ist.

8. Diazotypiematerial aus einem Träger mit einer lichtempfindlichen Schicht, die ein lichtempfindliches Benzoldiazoniumsalz gemäss Anspruch 1 enthält.

9. Einkomponenten-Diazotypiematerial aus einem Träger mit einer lichtempfindlichen Schicht, die ein lichtempfindliches Benzoldiazoniumsalz gemäss Anspruch 2 enthält.

10. Einkomponenten-Diazotypiematerial aus einem Träger mit einer lichtempfindlichen Schicht, die ein lichtempfindliches Benzoldiazoniumsalz gemäss Anspruch 3 enthält.

11. Einkomponenten-Diazotypiematerial aus einem Träger mit einer lichtempfindlichen Schicht, die ein lichtempfindliches Benzoldiazoniumsalz gemäss Anspruch 4 enthält.

12. Einkomponenten-Diazotypiematerial aus einem Träger mit einer lichtempfindlichen Schicht, die ein lichtempfindliches Benzoldiazoniumsalz gemäss Anspruch 5 enthält.

13. Einkomponenten-Diazotypiematerial aus einem Träger mit einer lichtempfindlichen Schicht, die ein lichtempfindliches Benzoldiazoniumsalz gemäss Anspruch 6 enthält.

14. Einkomponenten-Diazotypiematerial aus einem Träger mit einer lichtempfindlichen Schicht, die ein lichtempfindliches Benzoldiazoniumsalz gemäss Anspruch 7 enthält.

**Ansprüche** für den Vertragsstaat AT

1. Verwendung einer lichtempfindlichen Benzoldiazoniumverbindung der Formel

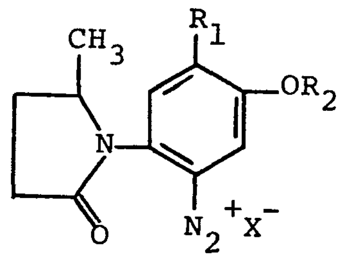

(I)

worin $R_1$ p-Methylphenylthio, Butylthio, Phenylthio oder Benzylthio, $R_2$ Methyl, Aethyl, Alkoxyalkyl mit 1 oder 2 Kohlenstoffatomen in dem Alkyl- und Alkoxyrest oder p-Chlorphenyl und X ein Anion ist, als Diazotypiematerial.

2. Verwendung einer Verbindung der Formel I nach Anspruch 1, worin X ein Chlor-, Tetrafluoroborat-, Hexafluorophosphat- oder $1/2ZnCl_4^-$-Ion ist, als Diazotypiematerial.

3. Verwendung einer Verbindung der Formel I nach Anspruch 1, worin $R_1$ p-Methylphenylthio, $R_2$ Methyl, Aethyl, Methoxyäthyl oder p-Chlorphenyl und X ein Chlor-, Tetrafluoroborat-, Hexafluorophosphat oder $1/2ZnCl_4^-$-Ion ist, als Diazotypiematerial.

4. Verwendung einer Verbindung der Formel I nach Anspruch 1, worin $R_1$ Butylthio, Phenylthio oder Benzylthio, $R_2$ Methyl und X ein Chlor-, Tetrafluoroborat-, Hexafluorophosphat- oder $1/2ZnCl_4^-$-Ion ist, als Diazotypiematerial.

5. Verwendung einer Verbindung der Formel I nach Anspruch 1, worin $R_1$ p-Methylphenylthio, $R_2$ Methyl, Aethyl, Methoxyäthyl oder p-Chlorphenyl und X ein $1/2ZnCl_4^-$-Ion ist, als Diazotypiematerial.

6. Verwendung einer Verbindung der Formel I nach Anspruch 1, worin $R_1$ Butylthio, Phenylthio oder Benzylthio, $R_2$ Methyl und X ein $1/2ZnCl_4^-$-Ion ist, als Diazotypiematerial.

7. Verwendung einer Verbindung der Formel I nach Anspruch 1, worin $R_1$ p-Methylphenylthio, $R_2$ Methyl und X ein $1/2ZnCl_4^-$-Ion ist, als Diazotypiematerial.

8. Diazotypiematerial aus einem Träger mit einer lichtempfindlichen Schicht, die ein lichtempfindliches Benzoldiazoniumsalz gemäss Anspruch 1 enthält.

9. Einkomponenten-Diazotypiematerial aus einem Träger mit einer lichtempfindlichen Schicht, die ein lichtempfindliches Benzoldiazoniumsalz gemäss Anspruch 2 enthält.

10. Einkomponenten-Diazotypiematerial aus einem Träger mit einer lichtempfindlichen Schicht, die ein lichtempfindliches Benzoldiazoniumsalz gemäss Anspruch 3 enthält.

11. Einkomponenten-Diazotypiematerial aus einem Träger mit einer lichtempfindlichen Schicht, die ein lichtempfindliches Benzoldiazoniumsalz gemäss Anspruch 4 enthält.

12. Einkomponenten-Diazotypiematerial aus einem Träger mit einer lichtempfindlichen Schicht, die ein lichtempfindliches Benzoldiazoniumsalz gemäss Anspruch 5 enthält.

13. Einkomponenten-Diazotypiematerial aus einem Träger mit einer lichtempfindlichen Schicht, die ein lichtempfindliches Benzoldiazoniumsalz gemäss Anspruch 6 enthält.

14. Einkomponenten-Diazotypiematerial aus einem Träger mit einer lichtempfindlichen Schicht, die ein lichtempfindliches Benzoldiazoniumsalz gemäss Anspruch 7 enthält.

**Claims** for the contracting states BE, CH, DE, FR, GB, IT, NL, SE

1. Photosensitive benzene diazonium compound of the formula

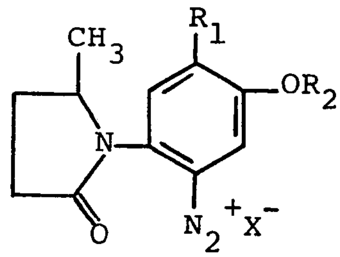

(I)

wherein $R_1$ is p-methylphenylthio, butylthio, phenylthio, or benzylthio, $R_2$ is methyl, ethyl, alkoxyalkyl having 1 or 2 carbon atoms in the alkyl and alkoxy radicals, or p-chloro-phenyl, and X is an anion.

2. Compound of formula I according to claim 1, wherein X is a chlorine, tetrafluoroborate, hexafluorophosphate, or $1/2ZnCl_4^-$ ion.

3. Compound of formula I according to claim 1, wherein $R_1$ is p-methylphenylthio, $R_2$ is methyl, ethyl, methoxyethyl, or p-chlorophenyl, and X is a chlorine, tetrafluoroborate, hexafluorophosphate, or $1/2ZnCl_4^-$ ion.

4. Compound of formula I according to claim 1, wherein $R_1$ is butylthio,phenylthio, or benzylthio, $R_2$ is methyl, and X is a chlorine, tetrafluoroborate, hexafluorophosphate, or $1/2ZnCl_4^-$ ion.

5. Compound of formula I according to claim 1, wherein $R_1$ is p-methylphenylthio, $R_2$ is methyl, ethyl, methoxyethyl, or p-chlorophenyl, and X is a $1/2ZnCl_4^-$ ion.

6. Compound of formula I according to claim 1, wherein $R_1$ is butylthio, phenylthio, or benzylthio, $R_2$ is methyl, and X is a $1/2ZnCl_4^-$ ion.

7. Compound of formula I according to claim 1, wherein $R_1$ is p-methylphenylthio, $R_2$ is methyl, and X is a $1/2ZnCl_4^-$ ion.

8. Diazotype material made of a support having a photosensitive layer containing a photosensitive benzene diazonium salt according to claim 1.

9. One-component diazotype material made of a support having a photosensitive layer containing a photosensitive benzene diazonium salt according to claim 2.

10. One-component diazotype material made of a support having a photosensitive layer containing a photosensitive benzene diazonium salt according to claim 3.

11. One-component diazotype material made of a support having a photosensitive layer containing a photosensitive benzene diazonium salt according to claim 4.

12. One-component diazotype material made of a support having a photosensitive layer containing a photosensitive benzene diazonium salt according to claim 5.

13. One-component diazotype material made of a support having a photosensitive layer containing a photosensitive benzene diazonium salt according to claim 6.

14. One-component diazotype material made of a support having a photosensitive layer containing a photosensitive benzene diazonium salt according to claim 7.

**Claims** for the contracting state AT

1. Use of a photosensitive benzene diazonium compound of the formula

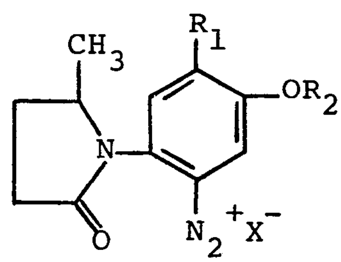

(I)

wherein $R_1$ is p-methylphenylthio, butylthio, phenylthio, or benzylthio, $R_2$ is methyl, ethyl, alkoxalkyl having 1 or 2 carbon atoms in the alkyl and alkoxy radicals, or p-chlorophenyl, and X is an anion as diazoptype material.

2. Use of a compound of formula I according to claim 1, wherein X is a chlorine, tetrafluoroborate, hexafluorophosphate, or $1/2ZnCl_4^-$ ion as diazoptype material.

3. Use of a compound of formula I according to claim 1, wherein $R_1$ is p-methylphenylthio, $R_2$ is methyl, ethyl, methoxyethyl, or p-chlorophenyl, and X is a chlorine, tetrafluoroborate hexafluorophosphate, or $1/2ZnCl_4^-$ ion as diazoptype material.

4. Use of a compound of formula I according to claim 1, wherein $R_1$ is butylthio, phenylthio, or benzylthio, $R_2$ is methyl, and X is a chlorine, tetrafluoroborate, hexafluorophosphate, or $1/2ZnCl_4^-$ ion as diazoptype material.

5. Use of compound of formula I according to claim 1, wherein $R_1$ is p-methylphenylthio, $R_2$ is methyl, ethyl, methoxyethyl, or p-chlorophenyl, and X is a $1/2ZnCl_4^-$ ion as diazoptype material.

6. Use of a compound of formula I according to claim 1, wherein $R_1$ is butylthio, phenylthio, or benzylthio, $R_2$ is methyl, and X is a $1/2ZnCl_4^-$ ion as diazoptype material.

7. Use of a compound of formula I according to claim 1, wherein $R_1$ is p-methylphenylthio, $R_2$ is methyl, and X is a $1/2ZnCl_4^-$ ion as diazoptype material.

8. Diazotype material made of a support having a photosensitive layer containing a photosensitive benzene diazonium salt according to claim 1.

9. One-component diazotype material made of a support having a photosensitive layer containing a photosensitive benzene diazonium salt according to claim 2.

10. One-component diazotype material made of a support having a photosensitive layer containing a photosensitive benzene diazonium salt according to claim 3.

11. One-component diazotype material made of a support having a photosensitive layer containing a photosensitive benzene diazonium salt accordig to claim 4.

12. One-component diazotype material made of a support having a photosensitive layer containing a photosensitive benzene diazonium salt according to claim 5.

13. One-component diazotype material made of a support having a photosensitive layer containing a photosensitive benzene diazonium salt according to claim 6.

14. One-component diazotype material made of a support having a photosensitive layer containing a photosensitive benzene diazonium salt according to claim 7.

**Revendications** pour les Etats contractants BE, CH, DE, FR, GB, IT, NL, SE

1. Composé de benzènediazonium photosensible de formule :

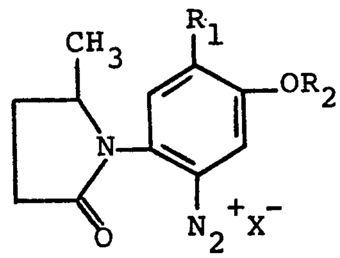

(I)

dans laquelle $R_1$ est un groupe p-méthylphénylthio, butylthio, phénylthio ou benzylthio, $R_2$ est un groupe méthyle, éthyle, alcoxyalcoyle ayant 1 ou 2 atomes de carbone dans la fraction alcoyle et alcoxy ou p-chlorophényle, et X est un anion.

2. Composé de formule I suivant la revendication 1, dans laquelle X est un ion chlore, tétrafluoborate, hexafluophosphate ou $1/2ZnCl_4^-$.

3. Composé de formule I suivant la revendication 1, dans laquelle $R_1$ est le groupe p-méthylphénylthio, $R_2$ est un groupe méthyle, éthyle, méthoxyéthyle, ou p-chlorophényle, et X est un ion chlore, tétrafluoborate, hexafluophosphate ou $1/2ZnCl_4^-$.

4. Composé de formule I suivant la revendication 1, dans laquelle $R_1$ est un groupe butylthio, phénylthio ou benzylthio, $R_2$ est le groupe méthyle, et X est un ion chlore, tétrafluoborate, hexafluophosphate ou $1/2ZnCl_4^-$.

5. Composé de formule I suivant la revendication 1, dans laquelle $R_1$ est le groupe p-méthylphénylthio, $R_2$ est un groupe méthyle, éthyle, méthoxyéthyle ou p-chlorophényle, et X est un ion $1/2ZnCl_4^-$.

6. Composé de formule I suivant la revendication 1, dans laquelle $R_1$ est un groupe butylthio, phénylthio ou benzylthio, $R_2$ est le groupe méthyle, et X est un ion $1/2ZnCl_4^-$.

7. Composé de formule I suivant la revendication 1, dans laquelle $R_1$ est le groupe p-méthylphénylthio, $R_2$ est le groupe méthyle et X est un ion $1/2ZnCl_4^-$.

8. Matériau pour diazotypie constitué par un support muni d'une couche photosensible qui renferme un sel de benzènediazonium photosensible suivant la revendication 1.

9. Matériau pour diazotypie formé d'un seul constituant, comprenant un support muni d'une couche photosensible qui renferme un sel de benzènediazonium photosensible suivant la revendication 2.

10. Matériau pour diazotypie formé d'un seul constituant, comprenant un support muni d'une couche photosensible qui renferme un sel de benzènediazonium photosensible suivant la revendication 3.

11. Matériau pour diazotypie formé d'un seul constituant, comprenant un support muni d'une couche photosensible qui renferme un sel de benzènediazonium photosensible suivant la revendication 4.

12. Matériau pour diazotypie formé d'un seul constitant, comprenant un support muni d'une couche photosensible qui renferme un sel de benzènediazonium photosensible suivant la revendication 5.

13. Matériau pour diazotypie formé d'un seul constituant, comprenant un support muni d'une couche photosensible qui renferme un sel de benzènediazonium photosensible suivant la revendication 6.

14. Matériau pour diazotypie formé d'un seul constituant, comprenant un support muni d'une couche photosensible qui renferme un sel de benzènediazonium photosensible suivant la revendication 7.

**Revendications** pour l'Etat contractant AT

1. Application d'un composé de benzènediazonium photosensible de formule :

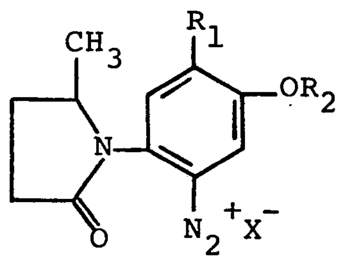

(I)

dans laquelle $R_1$ est un groupe p-méthylphénylthio, butylthio, phénylthio ou benzylthio, $R_2$ est un groupe méthyle, éthyle, alcoxyalcoyle ayant 1 ou 2 atomes de carbone dans la fraction alcoyle et alcoxy ou p-chlorophényle, et X est un anion, à titre de matériau pour diazotypie.

2. Application d'un composé de formule I suivant la revendication 1, dans laquelle X est un ion chlore, tétrafluoborate, hexafluophosphate ou $1/2ZnCl_4^-$, à titre de matériau pour diazotypie.

3. Application d'un composé de formule I suivant la revendication 1, dans laquelle $R_1$ est le groupe p-méthylphénylthio, $R_2$ est un groupe méthyle, éthyle, méthoxyéthyle ou p-chlorophényle, et X est un ion chlore, tétrafluoborate, hexafluophosphate ou $1/2ZnCl_4^-$, à titre de matériau pour diazotypie.

4. Application d'un composé de formule I suivant la revendication 1, dans laquelle $R_1$ est un groupe butylthio, phénylthio ou benzylthio, $R_2$ est le groupe méthyle, et X est un ion chlore, tétrafluoborate, hexafluophosphate ou $1/2ZnCl_4^-$, à titre de matériau pour diazotypie.

5. Application d'un composé de formule I suivant la revendication 1, dans laquelle $R_1$ est le groupe p-méthylphénylthio, $R_2$ est un groupe méthyle, éthyle, méthoxyéthyle ou p-chlorophényle, et X est un ion $1/2ZnCl_4^-$, à titre de matériau pour diazotypie.

6. Application d'un composé de formule I suivant la revendication 1, dans laquelle $R_1$ est un groupe butylthio, phénylthio ou benzylthio, $R_2$ est le groupe méthyle, et X est un ion $1/2ZnCl_4^-$, à titre de matériau pour diazotypie.

7. Application d'un composé de formule I suivant la revendication 1, dans laquelle $R_1$ est le groupe p-méthylphénylthio, $R_2$ est le groupe méthyle, et X est un ion $1/2ZnCl_4^-$, à titre de matériau pour diazotypie.

8. Matériau pour diazotypie constitué par un support muni d'une couche photosensible qui renferme un sel de benzènediazonium photosensible suivant la revendication 1.

9. Matériau pour diazotypie formé d'un seul constituant, comprenant un support muni d'une couche photosensible qui renferme un sel de benzènediazonium photosensible suivant la revendication 2.

10. Matériau pour diazotypie formé d'un seul constituant, comprenant un support muni d'une couche photosensible qui renferme un sel de benzènediazonium photosensible suivant la revendication 3.

11. Matériau pour diazotypie formé d'un seul constituant, comprenant un support muni d'une couche photosensible qui renferme un sel de benzènediazonium photosensible suivant la revendication 4.

12. Matériau pour diazotypie formé d'un seul constituant, comprenant un support muni d'une couche photosensible qui renferme un sel de benzènediazonium photosensible suivant la revendication 5.

13. Matériau pour diazotypie formé d'un seul constituant, comprenant un support muni d'une couche photosensible qui renferme un sel de benzènediazonium photosensible suivant la revendication 6.

14. Matériau pour diazotypie formé d'un seul constituant, comprenant un support muni d'une couche photosensible qui renferme un sel de benzènediazonium photosensible suivant la revendication 7.